(19) Europäisches Patentamt
European Patent Office
Office européen des brevets

(11) Numéro de publication: **0 015 219**
**B1**

(12) # FASCICULE DE BREVET EUROPEEN

(45) Date de publication du fascicule de brevet: **28.10.81**

(51) Int. Cl.³: **C 07 C 85/11**, C 07 C 85/24, C 07 C 87/60, C 07 C 93/14

(21) Numéro de dépôt: **80420018.6**

(22) Date de dépôt: **14.02.80**

(54) **Procédé de préparation d'anilines métachlorées.**

(30) Priorité: **15.02.79 FR 7904481**

(43) Date de publication de la demande: **03.09.80 Bulletin 80/18**

(45) Mention de la délivrance du brevet: **28.10.81 Bulletin 81/43**

(84) Etats contractants désignés: **AT BE CH DE FR GB IT LU NL SE**

(56) Documents cités: FR-A-2298531

(73) Titulaire: **RHONE-POULENC AGROCHIMIE, 14-20, rue Pierre Baizet, F-69009 Lyon (FR)**

(72) Inventeur: **Cordier, Georges, 50 Chemin des Hermières, F-69340 - Francheville (FR)**

(74) Mandataire: **Chretien, François et al, RHONE-POULENC AGROCHIMIE BP 9163, F-69263 Lyon Cedex 1 (FR)**

**EP 0 015 219 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG.

# Procédé de préparation d'anilines métachlorées

La présente invention se rapporte à un procédé de préparation d'anilines substituées par le chlore en position méta par action de l'hydrogène sur des composés aromatiques azotés plus fortement halogénés.

La préparation de chloranilines substituées en position méta par réaction de polychloranilines avec de l'hydrogène a été décrite dans le brevet français 2298531. Le procédé y décrit nécessite toutefois l'utilisation de pressions élevées et de quantités très importantes d'acide chlorhydrique, ce qui pose de graves problèmes de corrosion.

Un but de l'invention est de fournir un procédé permettant de préparer des anilines substituées en méta par le chlore avec de bons rendements à partir de composés aromatiques azotés plus fortement halogénés.

Un autre but de l'invention est de fournir un procédé de préparation d'anilines substituées en méta par le chlore, ce procédé pouvant mettre en œuvre comme réactif de départ aussi bien des composés chlorés nitrés (nitro-benzènes substitués et homologues) que aminés (polychloranilines et homologues).

Un autre but de l'invention est de fournir un procédé de préparation d'anilines métasubstituées par le chlore mettant en œuvre des pressions modérées.

Un autre but de l'invention est de fournir un procédé de préparation d'anilines métasubstituées par le chlore mettant en œuvre des températures de réaction modérées.

Un autre but, encore, de l'invention est de fournir un procédé de préparation d'anilines métasubstituées par le chlore mettant en œuvre des conditions modérément corrosives.

D'autres buts et avantages de l'invention apparaîtront au cours de la description qui va suivre.

Il a maintenant été trouvé que l'on pouvait atteindre ces buts grâce à un procédé de préparation d'anilines substituées, en position méta par du chlore, qui consiste à faire effectuer une hydrogénation catalytique en phase liquide, en milieu acide, à chaud, sous pression, de dérivés benzéniques azotés, en présence de métaux nobles du groupe VIII de la classification périodique, caractérisé en ce que les dérivés benzéniques ont la formule:

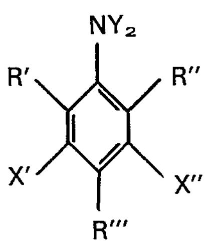

(I)

dans laquelle:

— Y représente l'atome d'hydrogène ou l'atome d'oxygène

— X' et X'', identiques ou différents entre eux, représentent chacun un atome de chlore ou un radical alkyle ou aryle, ou aralkyle ou alkoxyle ou aralkoxyle, éventuellement substitué, l'un des X' et X'' pouvant, de plus, être un atome d'hydrogène. Lorsqu'on prépare des monochloranilines (métachloranilines), un seul des substituants X' et X'' représente l'atome de chlore; lorsqu'on prépare des dichloranilines (dichlorées en méta), les deux symboles X' et X'' représentent l'atome de chlore.

— R', R'' et R''', identiques ou différents entre eux, représentent chacun un atome de chlore ou un radical alkyle ou aralkyle ou alkoxyle ou aryloxyle éventuellement substitué, l'un au moins de ces trois symboles représentant l'atome de chlore, au plus deux des R', R'' ou R''' pouvant, de plus, être l'hydrogène,

et en ce que la réaction est effectuée en présence d'ions iodure et/ou bromure.

La réaction s'effectue, comme il a été dit, en phase liquide; en pratique on opère avantageusement en présence d'un solvant minéral ou organique, liquide et inerte dans les conditions opératoires. Par solvant inerte on entend un solvant ne réagissant pas chimiquement. En fait on préfère utiliser l'eau.

L'acidité du milieu réactionnel est généralement telle que le pH (en cas de milieu aqueux) est avantageusement inférieur à 1,5, de préférence inférieur à 1. La concentration en ions $H^+$ dans le milieu est généralement comprise entre 0,5 et 12 ion-g/l, de préférence entre 1 et 6 ion-g/l.

Les concentrations les plus élevées en acide peuvent être utilisées mais sans avantage important.

L'acidité du milieu réactionnel peut être obtenue par des acides forts minéraux tels que les acides sulfurique, phosphorique ou halohydriques, ou organiques; mais l'on préfère l'utilisation des acides halohydriques et plus spécialement de l'acide chlorhydrique. De toute manière, étant donné la présence d'ions chlorure issus de la déshalogénation et d'ions iodure et/ou bromure caractéristiques de l'invention, on opère en fait toujours en présence, au moins partiellement, d'acides chlorhydrique, iodhydrique et/ou bromhydrique. Cependant, dans la mesure où ces acides sont dissociés dans le milieu, il n'est pas utile de définir les acides utilisés et il est préférable d'indiquer comme cela est fait ci-avant et ci-après la teneur en acidité (pH ou concentration en $H^+$) ainsi que la nature et la teneur en anions nécessaires à la réaction (anions halogénures).

Le procédé selon l'invention s'effectue en phase liquide (hormis bien sûr, le catalyseur à base de métal noble qui, le plus souvent, constitue une phase solide). La phase liquide peut être homogène et constituer une solution: c'est là une modalité préférée, spécialement lorsque Y est l'atome d'oxygène dans la formule (I); une telle phase liquide contient donc les réactifs, les produits de réaction et le ou les solvants éventuellement présents. Il est aussi possible d'opérer avec deux phases liquides.

La pression à laquelle s'effectue la réaction est généralement supérieure à 3 bars (pression relative) et de préférence supérieure à 5 bars. Il n'y a pas de limite supérieure critique pour la pression mais pour des raisons d'ordre économique il est généralement avantageux d'opérer à des pressions inférieures à 100 bars, les pressions inférieures à 30 bars étant préférées.

La température de réaction est généralement comprise entre 90 et 300°C, de préférence entre 110 et 200°C. Une température élevée peut, dans le cas où on utilise des acides relativement volatils, impliquer l'existence d'une pression partielle relativement importante pour les composés de la phase vapeur autres que l'hydrogène (par phase vapeur on entend évidemment la phase vapeur surmontant le milieu liquide réactionnel). On choisit généralement les conditions opératoires de manière que la pression partielle d'hydrogène soit comprise entre 10 et 80% de la pression totale (pression relative) et de préférence entre 30 et 60%.

Les métaux nobles constituant la base des catalyseurs utilisés dans l'invention sont principalement des métaux du groupe VIII de la classification périodique tels que le ruthénium, le rhodium, le palladium, l'osmium, l'iridium et le platine; le palladium est le métal préféré. Le métal peut être à l'état métallique ou à l'état de composé chimique; généralement on préfère que le métal soit mis en œuvre à l'état métallique car, dans les conditions opératoires, les composés ont tendance à être réduit à l'état métallique (degré d'oxydation=zéro). Le catalyseur peut être supporté ou non supporté. Comme support du catalyseur on peut utiliser tout support connu en soi pour supporter des catalyseurs, pourvu que ce support soit résistant à l'eau et aux acides; comme support convenant plus particulièrement, on peut citer le charbon actif, la silice, le sulfate de baryum; le charbon actif est un support préféré. Le catalyseur ainsi que son support sont avantageusement sous forme finement divisée; des surfaces spécifiques supérieures à 100 $m^2/g$ conviennent généralement bien.

La quantité de catalyseur mise en œuvre est telle que la proportion pondérale de métal noble du catalyseur par rapport au composé de formule (I) à traiter est généralement comprise entre 0,05 et 10%, de préférence entre 0,5 et 5%.

Les ions iodure et/ou bromure mis en œuvre dans la réaction peuvent être introduits sous les formes les plus diverses, notamment sous la forme d'acides iodhydrique et/ou bromhydrique, mais il est généralement plus commode de les introduire sous forme d'halogénures (iodures et/ou bromures) ou d'halohydrates (iodhydrates et/ou bromhydrates). Les iodures ou bromures sont habituellement des iodures ou bromures alcalins ou alcalino-terreux tels que les iodures ou bromures de lithium, sodium, potassium ou ammonium. Ces sels d'ammonium peuvent être des sels d'ammonium quaternaire ou non quaternaire (par exemple $NH_4^+$). Les iodhydrates ou bromhydrates sont de préférence des iodhydrates ou bromhydrates d'aniline substituée, telle que l'amine de formule (I) (Y=H) et/ou l'aniline substituée produite selon l'invention. Comme cela a déjà été dit, il se forme des ions chlorure au cours de la réaction en sorte que, sur un plan formel, on peut toujours estimer que la réaction se fait en présence d'acide chlorhydrique. La quantité d'ions iodure dans le milieu réactionnel est le plus souvent comprise entre $10^{-6}$ et 1 ion-g/l, de préférence entre 0,0001 et 0,1 ion-g/l. L'utilisation de faibles quantités d'ions iodure est spécialement avantageuse en ce qu'il est généralement superflu de les récupérer en fin de réaction. La quantité d'ions bromures dans le milieu réactionnel est le plus souvent comprise entre 0,01 et 10 ion-g/l, de préférence entre 0,1 et 6 ion-g/l.

Comme composés de formule (I) susceptibles d'être traités par le procédé de l'invention on peut citer de préférence:

le dichloro-2,3 nitrobenzène et la dichloro-2,3 aniline;

le dichloro-2,5 nitrobenzène et la dichloro-2,5 aniline;

le dichloro-3,4 nitrobenzène et la dichloro-3,4 aniline;

le trichloro-2,3,4 nitrobenzène et la trichloro-2,3,4 aniline;

le trichloro-2,3,5 nitrobenzène et la trichloro-2,3,5 aniline;

le trichloro-2,3,6 nitrobenzène et la trichloro-2,3,6 aniline;

le trichloro-2,4,5 nitrobenzène et la trichloro-2,4,5 aniline;

le trichloro-3,4,5 nitrobenzène et la trichloro-3,4,5 aniline;

le tétrachloro-2,3,4,6 nitrobenzène et la tétrachloro-2,3,4,6 aniline;

le tétrachloro-2,3,4,5 nitrobenzène et la tétrachloro-2,3,4,5 aniline;

le tétrachloro-2,3,5,6 nitrobenzène et la tétrachloro-2,3,5,6 aniline;

le pentachloro-nitrobenzène et la pentachloroaniline;

mais aussi:

le trichloro-4,5,6 méthyl-2 nitrobenzène et la trichloro-4,5,6 méthyl-2 aniline;

le dichloro-2,5 méthyl-4 nitrobenzène et la dichloro-2,5 méthyl-4 aniline;

le tétrachloro-2,3,5,6 méthyl-4 nitrobenzène et la tétrachloro-2,3,5,6 méthyl-4 aniline;

le dichloro-2,5 diméthyl-3,4 nitrobenzène et la dichloro-2,5 diméthyl-3,4 aniline;

le dichloro-2,5 éthyl-4 nitrobenzène et la dichloro-2,5 éthyl-4 aniline;

le dichloro-2,5 propyl-4 nitrobenzène et la dichloro-2,5 propyl-4 aniline;

le trichloro-3,4,6 benzyl-2 nitrobenzène et la trichloro-3,4,6 benzyl-2 aniline;

le dinitro-2,2' hexachloro-3,5,6,3',5',6' diphénylméthane et le diamino-2,2' hexachloro-3,5,6,3', 5', 6' diphénylméthane;

le nitro-2 trichloro-3,4,5 diphényle et l'amino-2 trichloro-3,4,5 diphényle;

le dinitro-4,4' octachloro diphényle et le diamino-4,4' octachloro diphényle;

le dichloro-4,5 méthoxy-2 nitrobenzène et la dichloro-4,5 méthoxy-2 aniline;
le dichloro-3,4 méthoxy-2 nitrobenzène et la dichloro-3,4 méthoxy-2 aniline;
le dichloro-3,6 méthoxy-2 nitrobenzène et la dichloro-3,6 méthoxy-2 aniline;
le dichloro-5,6 méthoxy-2 nitrobenzène et la dichloro-5,6 méthoxy-2 aniline;
le trichloro-3,4,6 méthoxy-2 nitrobenzène et la trichloro-3,4,6 méthoxy-2 aniline;
le trichloro-3,4,5 méthoxy-2 nitrobenzène et la trichloro-3,4,5 méthoxy-2 aniline;
le tétrachloro-3,4,5,6 méthoxy-2 nitrobenzène et la tétrachloro-3,4,5,6 méthoxy-2 aniline;
le dichloro-4,5 méthoxy-3 nitrobenzène et la dichloro-4,5 méthoxy-3 aniline;
le dichloro-5,6 méthoxy-3 nitrobenzène et la dichloro-5,6 méthoxy-3 aniline;
le dichloro-2,5 méthoxy-3 nitrobenzène et la dichloro-2,5 méthoxy-3 aniline;
le trichloro-4,5,6 méthoxy-3 nitrobenzène et la trichloro-4,5,6 méthoxy-3 aniline;
le tétrachloro-2,4,5,6 méthoxy-3 nitrobenzène et la tétrachloro-2,4,5,6 méthoxy-3 aniline;
le dichloro-2,3 méthoxy-4 nitrobenzène et la dichloro-2,3 méthoxy-4 aniline;
le dichloro-2,5 méthoxy-4 nitrobenzène et la dichloro-2,5 méthoxy-4 aniline;
le trichloro-2,3,6 méthoxy-4 nitrobenzène et la trichloro-2,3,6 méthoxy-4 aniline;
le trichloro-2,3,5 méthoxy-4 nitrobenzène et la trichloro-2,3,5 méthoxy-4 aniline;
le tétrachloro-2,3,5,6 méthoxy-4 nitrobenzène et la tétrachloro-2,3,5,6 méthoxy-4 aniline;
le dichloro-4,5 phénoxy-2 nitrobenzène et la dichloro-4,5 phénoxy-2 aniline;
le tétrachloro-3,4,5,6 phénoxy-2 nitrobenzène et la tétrachloro-3,4,5,6 phénoxy-2 aniline;
le tétrachloro-2,4,5,6 phénoxy-3 nitrobenzène et la tétrachloro-2,4,5,6 phénoxy-3 aniline;
le dichloro-2,5 phénoxy-4 nitrobenzène et la dichloro-2,5 phénoxy-4 aniline;
le tétrachloro-2,3,5,6 phénoxy-4 nitrobenzène et la tétrachloro-2,3,5,6 phénoxy-4 aniline.

Parmi les anilines substituées en position méta par l'atome de chlore et susceptibles d'être préparées par le procédé selon l'invention, on peut citer de préférence: la métachloraniline et la dichloro-3,5 aniline mais aussi la choro-5 méthyl-2 aniline; la chloro-5 méthyl-3 aniline; la chloro-3 méthyl-4 aniline; la dichloro-3,5 méthyl-4 aniline; la chloro-5 diméthyl-3,4 aniline; la chloro-3 éthyl-4 aniline; la chloro-3 benzyl-2 aniline; le diamino-4,4' tétrachloro-2,6,2',6' diphényle; la chloro-3 méthoxy-2 aniline; la chloro-5 méthoxy-2 aniline; la dichloro-3,5 méthoxy-2 aniline; la chloro-3 méthoxy-4 aniline; la chloro-5 méthoxy-3 aniline; la dichloro-3,5 méthoxy-4 aniline; la chloro-3 phénoxy-2 aniline; la chloro-5 phénoxy-2 aniline; la dichloro-3,5 phénoxy-2 aniline; la dichloro-3,5 phénoxy-4 aniline.

Le procédé selon l'invention peut être effectué en continu ou en discontinu. En fin de réaction le catalyseur peut être séparé, le cas échéant, par filtration ou par des moyens équivalents tels que l'essorage; l'amine métachlorée préparée peut être séparée par tout moyen connu en soi par exemple par extraction à l'aide d'un solvant et/ou par distillation; avant de procéder à cette séparation il convient généralement de faire repasser l'amine (salifiée en milieu acide) sous forme amine (non salifiée) par neutralisation ou alcalinisation à l'aide d'un agent alcalin. Dans le cas d'un procédé mettant en œuvre des ions bromure, il peut être avantageux de procéder à une récupération de ces ions, en vue d'un recyclage dans une opération ultérieure.

Le procédé selon l'invention est très avantageux en raison de sa bonne sélectivité en amine métachlorée et des conditions relativement douces qu'il permet de réaliser. Les amines métachlorées ainsi produites sont utilisables notamment pour fabriquer des pesticides.

Les exemples suivants, donnés à titre non limitatif, illustrent l'invention et montrent comment elle peut être mise en œuvre.

*Exemple 1*

Dans un autoclave de 250 $cm^3$ revêtu intérieurement de tantale, on charge:

— 1,67 g de tétrachloro-2,3,4,5 aniline,

— 0,07 g d'un catalyseur constitué de palladium déposé sur du charbon actif (surface spécifique du charbon actif: 1100 $m^2/g$; teneur pondérale en palladium: 10%),

— 106 $cm^3$ d'une solution aqueuse d'acide chlorhydrique de concentration 4 moles/l,

— 13,3 $cm^3$ d'une solution aqueuse d'acide iodhydrique à 7,6 moles/l.

L'autoclave est fermé, purgé d'abord à l'argon puis à l'hydrogène. On chauffe alors à 190°C en laissant croître la pression autogène, puis, lorsque cette température est atteinte, on introduit l'hydrogène jusqu'à une pression totale (relative) de 20 bars, dont 6 bars de pression partielle d'hydrogène.

On laisse réagir dans ces conditions pendant 1 heure. On refroidit; la masse réactionnelle liquide est alcalinisée par une solution aqueuse de soude (NaOH); le catalyseur est séparé par filtration; la dichloro-3,5 aniline est extraite de la phase aqueuse à l'aide de chlorure de méthylène; la solution dans le chlorure de méthylène ainsi obtenue est séchée sur sulfate de sodium; le solvant est évaporé sous vide; le taux de transformation de la tétrachloroaniline a été de 100% et le rendement en dichloro-3,5 aniline a été de 98,2%.

*Exemple 2*

On reproduit l'exemple 1 avec les modifications suivantes:

— on utilise 98 $cm^3$ d'une solution aqueuse d'acide chlorhydrique à 0,8 mole/l (au lieu de 106 $cm^3$ d'une solution à 4 moles/l),

— on utilise 63 g de NaI sec (au lieu de 13,3 $cm^3$ de solution de HI).

On obtient de la dichloro-3,5 aniline avec un rendement de 92,2%. Le taux de transformation de la tétrachloraniline est de 100%.

*Exemple 3*

On reproduit l'exemple 1 avec les modifications suivantes:

— on utilise 115 $cm^3$ d'une solution aqueuse d'acide chlorhydrique à 0,8 mole/l (au lieu de 106 $cm^3$ d'une solution à 4 moles/l),
— on utilise 14,8 g de NaI sec (au lieu de 13,3 $cm^3$ de solution de HI).

On obtient de la dichloro-3,5 aniline contenant 5,8% de trichloroaniline et 0,3% de métachloroaniline; le rendement en dichloroaniline est de 93,6%; le taux de transformation de la tétrachloroaniline est de 100%.

*Exemple 4*

On reproduit l'exemple 1 avec les modifications suivantes:
— on utilise 120 $cm^3$ de solution aqueuse d'acide chlorhydrique (au lieu de 106 $cm^3$),
— on utilise 0,4 $cm^3$ d'une solution aqueuse d'acide iodhydrique à 0,01 mole/l (au lieu de 13,3 $cm^3$ d'une solution à 7,6 moles/l),
— la réaction dure 5 heures (au lieu de 1 heure).

On obtient de la dichloro-3,5 aniline contenant 3,5% de métachloraniline avec un rendement de 96% en dichloro-3,5 aniline et un taux de transformation de la tétrachloroaniline de 100%.

*Exemple 5*

On reproduit l'exemple 1 avec les modifications suivantes:
— on charge 0,42 g de tétrachloroaniline (au lieu de 1,67 g),
— on charge 120 $cm^3$ de solution aqueuse d'acide chlorhydrique à 2,5 moles/l (au lieu de 106 $cm^3$ de solution à 4 moles/l),
— on charge 0,11 g de KI sec (au lieu de 13,3 $cm^3$ de solution de HI),
— la réaction dure 2 h 40 mn au lieu de 1 heure,
— la température est de 150°C (au lieu de 190°C),
— la pression est de 10 bars, dont 5 bars de pression partielle d'hydrogène.

On obtient de la dichloro-3,5 aniline contenant 2,5% d'aniline et 2,5% de métachloraniline avec un rendement en dichloro-3,5 aniline de 86%. Taux de transformation de la tétrachloroaniline: 100%.

*Exemple 6*

On reproduit l'exemple 1 avec les modifications suivantes:
— on utilise 1,17 g de dichloro-2,3 aniline (au lieu de la tétrachloroaniline),
— on utilise 0,56 g de catalyseur à base de palladium (au lieu de 0,07 g),
— on utilise 120 $cm^3$ de solution aqueuse d'HCl à 2,5 moles/l (au lieu de 106 $cm^3$ d'HCl à 4 moles/l),
— on ajoute 0,2 g de KI (au lieu de l'acide iodhydrique).

Le chauffage est effectué à 160°C; la pression totale est de 13 bars (dont 6 bars de pression partielle d'hydrogène); la durée de réaction est de 3 heures.

Le taux de transformation de la dichloro-2,3 aniline est de 98,2%; le rendement en métachloraniline est de 95,6% par rapport à la dichloroaniline transformée.

*Exemple 7*

On reproduit l'exemple 6 avec les modifications suivantes:
— on utilise la dichloro-3,4 aniline (au lieu de la dichloro-2,3 aniline),
— on utilise de l'acide chlorhydrique à 1 mole/l (au lieu de 2,5 moles/l),
— on utilise 0,04 g de KI (au lieu de 0,2 g),
— la durée de réaction est seulement de 45 mn.

La dichloro-3,4 aniline s'est complètement transformée; on obtient la métachloraniline avec un rendement de 97,1%.

*Exemple 8*

Dans un autoclave de 225 $cm^3$, on charge:
— 139 g de dichloro-3,4 nitrobenzène,
— 0,56 g de catalyseur (palladium sur charbon actif à 10% de palladium),
— 120 $cm^3$ d'une solution aqueuse d'acide chlorhydrique à 1 mole/l,
— 0,04 g de KI.

L'autoclave est purgé à l'aide d'argon puis d'hydrogène et on introduit l'hydrogène dans l'autoclave jusqu'à une pression de 4 bars. On porte la température de l'autoclave à 160°C. La pression est alors de 13 bars dont 6 bars de pression partielle d'hydrogène.

On laisse réagir 1 h 40 mn et on refroidit. Le taux de transformation du dichloronitrobenzène est de 100%. La métachloraniline a été obtenue avec un rendement de 92,9%.

*Exemple 9*

Dans un autoclave de 250 $cm^3$ revêtu intérieurement de tantale, on charge:
— 120 $cm^3$ d'une solution aqueuse d'acide chlorhydrique 4 N,
— 0,354 g de trichloro-3,4,5 aniline,
— 0,14 g de catalyseur à base de palladium tel qu'utilisé à l'exemple 1,
— 0,011 g de KI.

L'autoclave est fermé et purgé à l'argon puis à l'hydrogène. On chauffe pendant 2 h 30 mn à 130°C sous une pression totale de 8 bars et une pression partielle d'hydrogène de 5 bars. Au cours de la réaction la trichloraniline s'est transformée entièrement et on a obtenu la 3,5-dichloroaniline avec un rendement de 99%.

*Exemple 10*

Dans un autoclave de 250 $cm^3$ revêtu intérieurement de tantale on charge:
— 120 $cm^3$ d'une solution aqueuse d'HCl 4N,
— 3,54 g de trichloro 2-4-5 aniline,
— 1,4 g de catalyseur à base de palladium tel qu'utilisé dans les exemples précédents (palladium sur charbon actif à 10% de palladium),
— 0,011 g d'iodure de potassium.

L'autoclave est fermé et purgé à l'argon puis à l'hydrogène. On chauffe pendant 240 mn à 160°C sous une pression totale de 18 bars et une pression partielle d'hydrogène de 13 bars.

La transformation est complète et on obtient la 3 chloroaniline avec un rendement de 98,2%.

*Exemple 11*

On opère comme à l'exemple précédent sauf pour les conditions suivantes:

On charge:

— 120 $cm^3$ d'une solution aqueuse d'HCl 4N,

— 0,354 g de trichloro 2-3-5 aniline,

— 0,140 g de catalyseur à base de palladium (palladium sur charbon actif à 10% de palladium),

— 0,011 g d'iodure de potassium.

On chauffe pendant 160 mn à 160°C sous une pression totale de 18 bars dont 13 bars d'hydrogène.

La transformation est complète et on obtient la 3-5 dichloroaniline avec un rendement de 96,8%.

*Exemple 12*

On opère comme à l'exemple 1 du brevet sauf pour les conditions suivantes:

On charge:

— 120 $cm^3$ d'une solution aqueuse d'acide chlorhydrique à 4 moles/l,

— 1,67 g de 2-3-4-5 tétrachloroaniline,

— 0,07 g du catalyseur utilisé à l'exemple 1,

— 7,14 g de bromure de potassium.

On chauffe pendant 170 mn à 190°C sous une pression totale de 20 bars dont 6 bars de pression d'hydrogène.

La transformation est totale. On obtient la 3-5 dichloroaniline avec un rendement de 90%.

*Exemple 13*

On opère comme à l'exemple 1, sauf pour les conditions suivantes:

On charge:

— 120 ml d'acide chlorhydrique aqueux 4N,

— 420 mg de 2-3-4-5 tétrachloroaniline,

— 140 mg du catalyseur utilisé à l'exemple 1,

— 1,1 mg d'iodure de potassium (soit une concentration de $5{,}5\text{-}10^{-5}$ dans le milieu).

On chauffe pendant 220 mn à 160°C sous une pression totale de 9 bars dont 4,5 bars d'hydrogène.

La transformation de la tétrachloraniline est complète. On obtient la 3-5 dichloroaniline avec un rendement de 92,5%.

*Exemple 14*

On opère comme à l'exemple 1, sauf pour les conditions suivantes:

On charge:

— 120 ml d'acide chlorhydrique aqueux 4N,

— 420 mg de 2-3-4-5 tétrachloroaniline,

— 140 mg de catalyseur à 1,1% de palladium déposé sur charbon actif,

— 11 g d'iodure de potassium.

On chauffe pendant 180 mn à 160°C sous pression totale de 18 bars dont 13 d'hydrogène. On obtient la 3-5 dichloroaniline avec un rendement de 98,1%. La transformation de la tétrachloroaniline est complète.

*Exemple 15*

Le même résultat qu'à l'exemple 14 est obtenu avec les mêmes charges et les mêmes conditions en remplaçant les 140 mg de catalyseur à 1,1% de palladium par 140 mg d'un catalyseur à 2,8% de palladium déposé sur le même support par la même méthode.

*Exemple 16*

Dans un autoclave de 3,6 l revêtu intérieurement de tantale, on charge:

— 2 kg d'une solution aqueuse d'acide chlorhydrique à 13,6%,

— 150 g de catalyseur constitué de palladium déposé sur charbon actif (surface spécifique du charbon actif: 1100 $m^2/g$; teneur pondérale en palladium: 10%),

— 11,7 g d'iodure de potassium sec et solide soit 0,5 ion/g d'ion iodure par atome gramme de palladium.

L'autoclave est fermé, purgé d'abord à l'azote puis à l'hydrogène. On injecte alors de l'hydrogène jusqu'à une pression de 3 bars. On chauffe à 160°C en laissant croître la pression autogène. On injecte à nouveau de l'hydrogène pour que la pression s'établisse à 15,5 bars dont 10 bars de pression partielle d'hydrogène. Puis, on injecte 1 mole de 2-3-4-5 tétrachloronitrobenzène en 2 h 30 mn.

On laisse réagir dans ces conditions et sous agitation pendant 2 h. On refroidit à 100°C puis on vide le réacteur; le milieu réactionnel liquide est neutralisé à la soude aqueuse; le catalyseur est séparé par filtration, les amines chlorées, principalement la 3,5 dichloroaniline, sont extraites au toluène. Le solvant est évaporé sous vide.

Dans ces conditions, le taux de transformation du 2-3-4-5 tétrachlorobenzène est de 100% et le rendement en 3,5 dichloroaniline de 94%.

**Revendications**

1. Procédé de préparation d'anilines substituées en position méta par du chlore, par hydrogénation catalytique en phase liquide, en milieu acide, à chaud et sous pression de dérivés benzéniques azotés et chlorés, en présence de métaux nobles du groupe VIII de la classification périodique, caractérisé en ce que les dérivés benzéniques ont la formule:

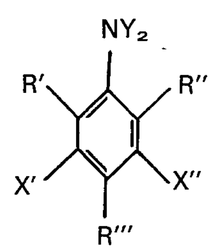

(I)

dans laquelle

— Y représente l'atome d'hydrogène ou l'atome d'oxygène,

— X' et X'', identiques ou différents entre eux, représentent chacun un atome de chlore ou un radical alkyle ou aryle ou aralkyle ou alkoxyle ou aralkoxyle éventuellement substitué, l'un des X' et X'' pouvant, de plus être l'hydrogène,

— R', R'' et R''', identiques ou différents entre eux, représentent chacun un atome de chlore ou un radical alkyle ou aralkyle ou alkoxyle ou aryloxyle

éventuellement substitué, l'un au moins de ces trois symboles représentant l'atome de chlore, au plus deux des R', R'' et R''' pouvant de plus être l'hydrogène, et en ce que la réaction est effectuée en présence d'ions iodure et/ou bromure.

2. Procédé selon la revendication 1, caractérisé en ce que R', R'', R''', X', X'', identiques ou différents entre eux, représentent l'atome d'hydrogène ou l'atome de chlore.

3. Procédé de préparation de dimétachloroanilines, éventuellement substituées, selon l'une des revendications 1 ou 2 caractérisé en ce que X' et X'' représentent l'atome de chlore.

4. Procédé de préparation de monométachloroanilines, éventuellement substituées, selon l'une des revendications 1 et 2 caractérisé en ce que un seul des deux radicaux X' et X'' est l'atome de chlore.

5. Procédé de préparation de dichloro-3,5 aniline selon les revendications 1 à 3 caractérisé en ce que Y est l'atome d'hydrogène ou d'oxygène, X' et X'' sont l'atome de chlore, R', R'', R''' sont l'atome d'hydrogène ou l'atome de chlore, l'un d'entre eux au moins étant l'atome de chlore.

6. Procédé selon l'une des revendications 1 à 5, caractérisé en ce que le pH est inférieur à 1,5 et/ou que la concentration en ions $H^+$ du milieu réactionnel est comprise entre 0,5 et 12 ion−g/l.

7. Procédé selon la revendication 6, caractérisé en ce que le pH est inférieur à 1 et/ou que la concentration en ions $H^+$ du milieu réactionnel est comprise entre 1 et 6 ion−g/l.

8. Procédé selon l'une des revendications 1 à 7, caractérisé en ce que le milieu réactionnel est un milieu aqueux.

9. Procédé selon l'une des revendications 1 à 8, caractérisé en ce que le milieu réactionnel ne comporte qu'une phase liquide, hormis le catalyseur à base de métal noble.

10. Procédé selon l'une des revendications 1 à 9, caractérisé en ce que la pression totale est comprise entre 3 et 100 bars.

11. Procédé selon la revendication 10, caractérisé en ce que la pression totale est comprise entre 5 et 30 bars.

12. Procédé selon l'une des revendications 1 à 11, caractérisé en ce que la température est comprise entre 90 et 300°C, de préférence entre 110 et 200°C.

13. Procédé selon l'une des revendications 1 à 12, caractérisé en ce que la pression partielle d'hydrogène est comprise entre 10 et 80% de la pression totale.

14. Procédé selon la revendication 13, caractérisé en ce que la pression partielle d'hydrogène est comprise entre 30 et 60% de la pression totale.

15. Procédé selon les revendications 1 à 14, caractérisé en ce que le catalyseur est du palladium.

16. Procédé selon l'une des revendications 1 à 15, caractérisé en ce que la proportion pondérale de métal noble par rapport au composé de formule (I) est comprise entre 0,05 et 10%, de préférence entre 0,5 et 5%.

17. Procédé selon l'une des revendications 1 à 16, caractérisé en ce que la proportion d'ions iodure dans le milieu réactionnel est comprise entre $10^{-6}$ et 1 ion-g/l et/ou que la proportion d'ions bromure dans le milieu réactionnel est comprise entre 0,01 et 10 ion-g/l.

18. Procédé selon la revendication 17, caractérisé en ce que la proportion d'ions iodure dans le milieu réactionnel est comprise entre 0,0001 et 0,1 ion-g/l et/ou que la proportion d'ions bromure est comprise entre 0,1 et 5 ion-g/l.

**Ansprüche**

1. Verfahren zur Herstellung von m-chlorsubstituierten Anilinen durch katalytische Hydrierung von stickstoffhaltigen und chlorierten Benzolderivaten in flüssiger Phase in einem sauren Medium in der Wärme und unter Druck in Gegenwart von Edelmetallen der Gruppe VIII des Periodensystems,
dadurch gekennzeichnet, dass
als Benzolderivate Verbindungen der Formel I

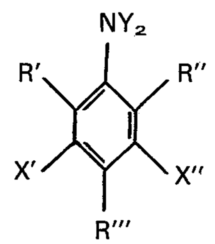

(I)

verwendet werden, in der bedeuten:
Y Wasserstoff oder Sauerstoff,
X' und X'' zugleich oder unabhängig Chlor, Alkyl, Aryl, Aralkyl, Alkoxy oder Aralkoxy, die ggf substituiert sind, wobei ferner einer der beiden Substituenten X' und X'' auch Wasserstoff bedeuten kann,
und
R', R'' und R''' zugleich oder unabhängig Chlor, Alkyl, Aralkyl, Alkoxy oder Aryloxy, die ggf substituiert sind, wobei mindestens einer der Substituenten R', R'' und R''' Chlor ist und ferner höchstens zwei dieser Substituenten Wasserstoff bedeuten können,
und
die Reaktion in Gegenwart von Jodidionen und/ oder Bromidionen durchgeführt wird.

2. Verfahren nach Anspruch 1, dadurch gekennzeichnet, dass ein Benzolderivat der Formel I verwendet wird, in der R', R'', R''', X' und X'' zugleich oder unabhängig Wasserstoff oder Chlor bedeuten.

3. Verfahren nach Anspruch 1 oder 2 zur Herstellung ggf substituierter Di-meta-chloraniline, dadurch gekennzeichnet, dass eine Verbindung der Formel I eingesetzt wird, in der X' und X'' Chlor bedeuten.

4. Verfahren nach Anspruch 1 oder 2 zur Herstellung ggf substituierter Mono-meta-chloraniline, dadurch gekennzeichnet, dass eine Verbindung der Formel I verwendet wird, in der nur einer der Substituenten X' und X'' Chlor bedeutet.

5. Verfahren nach einem der Ansprüche 1 bis 3 zur Herstellung von 3,5-Dichloranilin, dadurch

gekennzeichnet, dass eine Verbindung der Formel I verwendet wird, in der bedeuten:
Y Wasserstoff oder Sauerstoff,
X' und X'' Chlor und
R', R'' und R''' Wasserstoff oder Chlor, wobei mindestens einer der Substituenten R', R'' und R''' Chlor bedeutet.

6. Verfahren nach einem der Ansprüche 1 bis 5, dadurch gekennzeichnet, dass bei einem pH-Wert des Reaktionsmediums unter 1,5 und/oder einer $H^+$-Ionenkonzentration des Reaktionsmediums zwischen 0,5 und 12 val $H^+$/l gearbeitet wird.

7. Verfahren nach Anspruch 6, dadurch gekennzeichnet, dass bei einem pH-Wert des Reaktionsmediums unter 1 und/oder einer $H^+$-Ionenkonzentration des Reaktionsmediums von 1 bis 6 val $H^+$/l gearbeitet wird.

8. Verfahren nach einem der Ansprüche 1 bis 7, dadurch gekennzeichnet, dass als Reaktionsmedium ein wässriges Medium verwendet wird.

9. Verfahren nach einem der Ansprüche 1 bis 8, dadurch gekennzeichnet, dass das Reaktionsmedium, abgesehen vom Edelmetallkatalysator, aus lediglich einer flüssigen Phase besteht.

10. Verfahren nach einem der Ansprüche 1 bis 9, dadurch gekennzeichnet, dass ein Gesamtdruck von 3 bis 100 bar angewandt wird.

11. Verfahren nach Anspruch 10, dadurch gekennzeichnet, dass ein Gesamtdruck von 5 bis 30 bar angewandt wird.

12. Verfahren nach einem der Ansprüche 1 bis 11, dadurch gekennzeichnet, dass bei einer Temperatur von 90 bis 300°C und vorzugsweise von 110 bis 200°C verfahren wird.

13. Verfahren nach einem der Ansprüche 1 bis 12, dadurch gekennzeichnet, dass bei einem Wasserstoffpartialdruck von 10 bis 80% des Gesamtdrucks gearbeitet wird.

14. Verfahren nach Anspruch 13, dadurch gekennzeichnet, dass bei einem Wasserstoffpartialdruck von 30 bis 60% des Gesamtdrucks gearbeitet wird.

15. Verfahren nach einem der Ansprüche 1 bis 14, dadurch gekennzeichnet, dass als Katalysator Palladium verwendet wird.

16. Verfahren nach einem der Ansprüche 1 bis 15, dadurch gekennzeichnet, dass das Edelmetall des Katalysators in einer Menge von 0,05 bis 10 Gew.%, vorzugsweise von 0,5 bis 5 Gew.% bezogen auf das Gewicht der Verbindung der Formel I, eingesetzt wird.

17. Verfahren nach einem der Ansprüche 1 bis 16, dadurch gekennzeichnet, dass die Menge der im Reaktionsmedium eingesetzten Jodidionen $10^{-6}$ bis 1 val/l und/oder die Menge der im Reaktionsmedium eingesetzten Bromidionen $10^{-2}$ bis 10 val/l beträgt.

18. Verfahren nach Anspruch 17, dadurch gekennzeichnet, dass die Menge der im Reaktionsmedium eingesetzten Jodidionen $10^{-4}$ bis $10^{-1}$ val/l und/oder die Menge der eingesetzten Bromidionen $10^{-1}$ bis 5 val/l beträgt.

**Claims**

1. A process for the preparation of anilines substituted in the meta-position by chlorine, by the catalytic hydrogenation of chlorine-substituted nitrogen-containing benzene derivatives, in the liquid phase, in an acid medium, under the action of heat, under pressure and in the presence of noble metals from group VIII of the periodic classification, in which process the benzene derivatives have the formula:

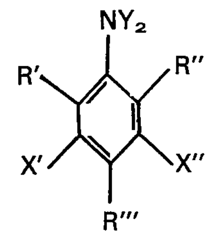

(I)

in which Y represents the hydrogen atom or the oxygen atom, X' and X'', which are identical or different from one another, each represent a chlorine atom or an optionally substituted alkyl, aryl, aralkyl, alkoxy or aralkoxy radical, it being furthermore possible for one of the symbols X' and X'' to be hydrogen, and R', R'' and R''', which are identical or different from one another, each represent a chlorine atom or an optionally substituted alkyl, aralkyl, alkoxy or aryloxy radical, at least one of these three symbols representing the chlorine atom and it being furthermore possible for at most two of the symbols R', R'' and R''' to be hydrogen, and the reaction is carried out in the presence of iodide and/or bromide ions.

2. A process according to claim 1, in which R', R'', R''', X' and X'', which are identical or different from one another, represent the hydrogen atom or the chlorine atom.

3. A process for the preparation of optionally substituted meta-dichloroanilines, according to one of claims 1 or 2, in which X' and X'' represent the chlorine atom.

4. A process for the preparation of optionally substituted meta-monochloroanilines, according to one of claims 1 or 2, in which only one of the two radicals X' and X'' is the chlorine atom.

5. A process for the preparation of 3,5-dichloroaniline, according to claims 1 to 3, in which Y is the hydrogen or oxygen atom, X' and X'' are the chlorine atom and R', R'' and R''' are the hydrogen atom or the chlorine atom, at least one of them being the chlorine atom.

6. A process according to one of claims 1 to 5, in which the pH is less than 1.5 and/or the concentration of $H^+$ ions in the reaction medium is between 0.5 and 12 g.ions/l.

7. A process according to claim 6, in which the pH is less than 1 and/or the concentration of $H^+$ ions in the reaction medium is between 1 and 6 g.ions/l.

8. A process according to one of claims 1 to 7, in which the reaction medium is an aqueous medium.

9. A process according to one of claims 1 to 8, in which the reaction medium consists only of a

liquid phase, except for the catalyst based on a noble metal.

10. A process according to one of claims 1 to 9, in which the total pressure is between 3 and 100 bars.

11. A process according to claim 10, in which the total pressure is between 5 and 30 bars.

12. A process according to one of claims 1 to 11, in which the temperature is between 90 and 300°C and preferably between 110 and 200°C.

13. A process according to one of claims 1 to 12, in which the hydrogen partial pressure is between 10 and 80% of the total pressure.

14. A process according to claim 13, in which the hydrogen partial pressure is between 30 and 60% of the total pressure.

15. A process according to claims 1 to 14, in which the catalyst is palladium.

16. A process according to one of claims 1 to 15, in which the proportion by weight of noble metal, relative to the compound of the formula (I), is between 0.05 and 10% and preferably between 0.5 and 5%.

17. A process according to one of claims 1 to 16, in which the proportion of iodide ions in the reaction mediums is between $10^{-6}$ and 1 g.ion/l and/or the proportion of bromide ions in the reaction medium is between 0.01 and 10 g.ions/l.

18. A process according to claim 17, in which the proportion of iodide ions in the reaction medium is between 0.0001 and 0.1 g.ion/l and/or the proportion of bromide ions is between 0.1 and 5 g.ions/l.